# EUROPEAN PATENT APPLICATION

(11) **EP 4 606 903 A1**
(43) Date of publication of application: **27.08.2025**
(21) Application number: 23878547.1
(22) Date of filing: 22.03.2023
(51) Int. Cl.: C12P 19/38, C12N 9/10

(54) **METHOD FOR ENZYMATIC SYNTHESIS OF NUCLEOSIDE CONTAINING PROTECTING GROUP, AND COMPOSITION**

(30) Priority: 21.10.2022 CN 202211296608
(71) Applicant: Asymchem Life Science (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); LI, Mingji, Tianjin 300457 (CN); LI, Rui, Tianjin 300457 (CN); DING, Shimao, Tianjin 300457 (CN)
(74) Representative: CAPRI
(86) International application number: PCT/CN2023/083182
(87) International publication number: WO 2024/082545

(57) **Abstract**

The present application provides a method for enzymatic synthesis of a nucleoside bearing a protecting group, and a composition. The method includes: using pyrimidine nucleoside phosphorylase or uracil nucleoside phosphorylase and purine nucleoside phosphorylase to catalyze a substrate to synthesize a nucleoside bearing a protecting group, where the substrate includes a substrate nucleoside, a substrate base and a substrate phosphate, and the substrate base bears a protecting group; the pyrimidine nucleoside phosphorylase includes a protein that is of more than 80% identity to a protein PyNP shown in SEQ ID NO: 1; the uracil nucleoside phosphorylase includes a protein that is of more than 80% identity to a protein UP shown in SEQ ID NO: 2; and the purine nucleoside phosphorylase includes a protein that is of more than 80% identity to a protein PNP shown in SEQ ID NO: 3, 7 or 8. The present application can solve the problem that there is no biosynthesis method to produce a nucleoside bearing a protecting group in the prior art, and thus is suitable for the field of enzyme catalysis.

## Description

This application is based upon and claims priority to Chinese Patent Application No. 202211296608.4 filed on October 21, 2022, the application of which is hereby incorporated by reference in its entirety.

### Technical Field

The present application relates to the field of enzyme catalysis, and specifically, to a method for enzymatic synthesis of a nucleoside bearing a protecting group, and a composition.

### Background

Nucleic acid drugs include antisense oligonucleotides and DNA aptamers, which may be used to treat diseases such as eye symptoms, and are one of the hot areas for new drug development in recent years. With the development and research of the drugs, the demand for its synthetic precursor nucleoside monomers in the drug market is increasing day by day. Currently, the nucleoside monomers are mainly prepared by a chemical synthesis method. Since a nucleoside carries a plurality of functional groups such as amino, hydroxyl, etc., some functional groups on a base are often required to be protected to selectively react at specific sites such as a 5'-position of 2'-deoxyribose for efficient DNA synthesis. In addition, these protecting groups may be easily removed under relatively-mild conditions, thereby obtaining products with high purity.

Commonly-used protecting groups include benzoyl, isobutyryl, acetyl, etc. For example, adenine may be protected with the benzoyl, guanine is protected with the isobutyryl, and cytosine may be protected with the benzoyl or the acetyl. The synthesis of nucleosides with base protection has been less reported and also achieved mainly by chemical methods. Patent application WO0075154A2 disclosed a nucleoside with base protection prepared by a chemical method, including N6-benzoyl-2'-deoxyadenosine, N2-isobutyryl-2'-deoxyguanosine, N4-benzoyl-2'-deoxycytidine, and the like. Patent application US2003162957A1 disclosed a N2-isobutyryl-2'-deoxyguanosine prepared by a chemical method. At present, there are no reports of a biosynthesis method to produce nucleosides bearing a protecting group.

### Summary

The present application is mainly intended to provide a method for enzymatic synthesis of a nucleoside bearing a protecting group, and a composition, so as to solve the problem that there is no biosynthesis method to produce a nucleoside bearing a protecting group in the prior art.

In order to implement the above objective, a first aspect of the present application provides a method for enzymatic synthesis of a nucleoside bearing a protecting group. The method includes: using a purine nucleoside phosphorylase and any one of following: a pyrimidine nucleoside phosphorylase or a uridine phosphorylase, to catalyze a substrate so as to synthesize a nucleoside bearing a protecting group, where the substrate includes a substrate nucleoside, a substrate base, and a substrate phosphate, and the substrate base bears the protecting group; the pyrimidine nucleoside phosphorylase includes PyNP, or a protein that is of more than 80% identity to the PyNP and has the same function, and the PyNP is a protein with the amino acid sequence shown as SEQ ID NO: 1; the uridine phosphorylase includes UP, or a protein that is of more than 80% identity to the UP and has the same function, and the UP is a protein with the amino acid sequence shown as SEQ ID NO: 2; and the purine nucleoside phosphorylase includes PNP, or a protein that is of more than 80% identity to the PNP and has the same function, and the PNP includes a protein with the amino acid sequence shown as SEQ ID NO: 3, SEQ ID NO: 7 or SEQ ID NO: 8.

Further, the protecting group is located on a base structure of the nucleoside. Preferably, the method includes: catalyzing the substrate nucleoside and the substrate phosphate with the pyrimidine nucleoside phosphorylase or the uracil nucleoside phosphorylase to generate a phosphate pentose and a free base; and substituting a phosphate group of the phosphate pentose which the substrate base bears the protecting group with the purine nucleoside phosphorylase, so as to obtain the nucleoside bearing the protecting group.

Further, the substrate nucleoside is a nucleoside shown in a Formula I, where R₁ is selected from -H or -OH or -F, and R₂ is selected from -H or -CH₃; preferably, the substrate nucleoside comprises thymidine, 2'-deoxyuridine, uridine or 1-beta-D-arabinofuranosyluracil; and further, the substrate base is a base shown in a Formula II, a Formula III or a Formula IV, where X, Y and Z are each independently selected from

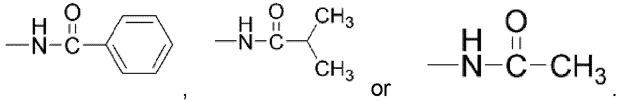

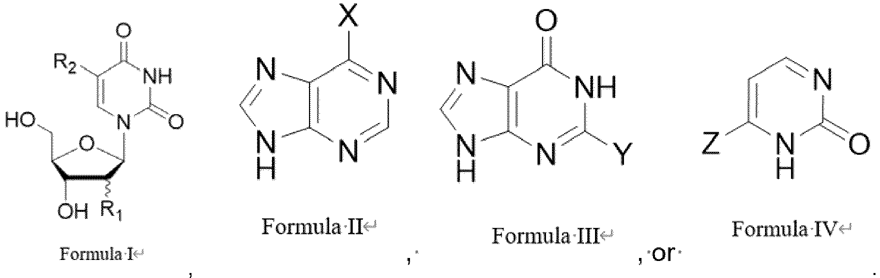

Preferably, the substrate base includes N6-benzoyladenine, N2-isobutyrylguanine or N4-acetylcytosine.

Further, one or more of the purine nucleoside phosphorylase, the pyrimidine nucleoside phosphorylase or the uracil nucleoside phosphorylase are purified proteins, crude enzyme liquid or immobilized enzymes.

Further, a catalytic time for the enzymatic synthesis is 4-20 h; preferably, a catalytic temperature of the enzymatic synthesis is 50-70 °C, more preferably, 60 °C; and preferably, a concentration of the substrate nucleoside is 20-400 mM, a concentration of the substrate base is 10-200 mM, and a concentration of the substrate phosphate is 1-100 mM.

Further, the nucleoside bearing the protecting group includes N6-benzoyladenosine, N2-isobutyrylguanosine, N4-acetylcytidine, N6-benzoyl-2'-deoxyadenosine, N2-isobutyryl-2'-deoxyguanosine, N4-acetyl-2'-deoxycytidine, N6-benzoyl-arabinosyladenosine, or N2-isobutyryl-arabinosylguanosine.

In order to implement the above objective, a second aspect of the present application provides a composition. The composition includes any one of the following enzymes: a pyrimidine nucleoside phosphorylase or a uracil nucleoside phosphorylase, and a purine nucleoside phosphorylase. The pyrimidine nucleoside phosphorylase includes PyNP, or a protein that is of more than 80% identity to the PyNP and has the same function, and the PyNP is a protein with the amino acid sequence shown as SEQ ID NO: 1; the uridine phosphorylase includes UP, or a protein that is of more than 80% identity to the UP and has the same function, and the UP is a protein with the amino acid sequence shown as SEQ ID NO: 2; and the purine nucleoside phosphorylase includes PNP, or a protein that is of more than 80% identity to the PNP and has the same function, and the PNP includes a protein with the amino acid sequence shown as SEQ ID NO: 3, SEQ ID NO: 7 or SEQ ID NO: 8.

Further, one or more of the purine nucleoside phosphorylase, the pyrimidine nucleoside phosphorylase or the uracil nucleoside phosphorylase are purified proteins, crude enzyme liquid or immobilized enzymes.

Further, the composition further includes a substrate nucleoside and a substrate base. The substrate includes the substrate nucleoside and the substrate base. The substrate base bears a protecting group. The substrate nucleoside is a nucleoside shown in a Formula I, where R₁ is selected from -H or -OH or -F, and R₂ is selected from -H or -CH₃. Preferably, the substrate nucleoside includes thymidine, 2'-deoxyuridine, uridine or 1-beta-D-arabinofuranosyluracil. Preferably, the substrate base is a base shown in a Formula II, a Formula III or a Formula IV, where X, Y and Z are each independently selected from 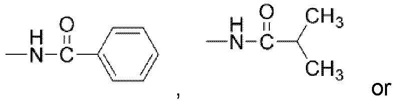 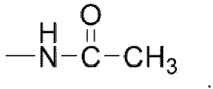 Preferably, the substrate base includes N6-benzoyladenine, N2-isobutyrylguanine or N4-acetylcytosine. Preferably, a concentration of the substrate nucleoside is 20-400 mM, a concentration of the substrate base is 10-200 mM, and a concentration of the substrate phosphate is 1-100 mM.

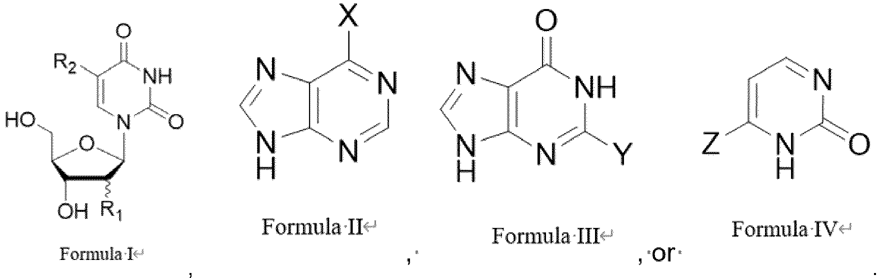

Through the application of the technical solutions of the present application, with the purine nucleoside phosphorylase and the pyrimidine nucleoside phosphorylase or the uracil nucleoside phosphorylase, enzymatic catalysis can be performed with the substrate nucleoside and the substrate base bearing the protecting group, so as to synthesize the nucleoside bearing the protecting group with a biosynthesis method.

### Brief Description of the Drawings

The drawings, which form a part of the present application, are used to provide a further understanding of the present application. The exemplary embodiments of the present application and the description thereof are used to explain the present application, but do not constitute improper limitations to the present application. In the drawings:
Fig. 1 is an HPLC diagram of synthesis of N6-benzoyladenosine with uridine as a substrate according to Embodiment 2 of the present application.
Fig. 2 is an HPLC diagram of synthesis of N2-isobutyrylguanosine with uridine as a substrate according to Embodiment 3 of the present application.
Fig. 3 is an HPLC diagram of synthesis of N4-acetylcytidine with uridine as a substrate according to Embodiment 4 of the present application.
Fig. 4 is an HPLC diagram of synthesis of N6-benzoyl-2'-deoxyadenosine with 2'-deoxyuridine as a substrate according to Embodiment 5 of the present application.
Fig. 5 is an HPLC diagram of synthesis of N2-isobutyryl-2'-deoxyguanosine with 2'-deoxyuridine as a substrate according to Embodiment 6 of the present application.
Fig. 6 is an HPLC diagram of synthesis of N4-acetyl-2'-deoxycytidine with 2'-deoxyuridine as a substrate according to Embodiment 7 of the present application.
Fig. 7 is an HPLC diagram of synthesis of N6-benzoyl-2'-deoxyadenosine with thymidine as a substrate according to Embodiment 8 of the present application.
Fig. 8 is an HPLC diagram of synthesis of N2-isobutyryl-2'-deoxyguanosine with thymidine as a substrate according to Embodiment 9 of the present application.
Fig. 9 is an HPLC diagram of synthesis of N6-benzoyl-2'-deoxyadenosine with thymidine as a substrate according to Embodiment 10 of the present application.
Fig. 10 is an HPLC diagram of synthesis of N4-acetyl-2'-deoxycytidine with thymidine as a substrate according to Embodiment 11 of the present application.
Fig. 11 is an HPLC diagram of synthesis of N6-benzoyl-arabinosyladenosine with 1-beta-D-arabinofuranosyluracil as a substrate according to Embodiment 12 of the present application.
Fig. 12 is an HPLC diagram of synthesis of N2-isobutyryl-arabinosylguanosine with 1-beta-D-arabinofuranosyluracil as a substrate according to Embodiment 13 of the present application.

### Detailed Description of the Embodiments

It is to be noted that the embodiments in the present application and the features in the embodiments may be combined with one another without conflict. The present application will be described below in detail with reference to the embodiments.

As mentioned in the Background, a nucleoside bearing a protecting group (e.g. the nucleoside bearing a protecting group on the base) in the prior art is prepared basically by means of chemical synthesis, and chemical synthesis is often accompanied by harsh reaction conditions and high levels of by-products and pollutants. Therefore, in the present application, the inventor attempts to develop a method for enzymatic synthesis of a nucleoside bearing a protecting group, and a composition, so as to prepare a nucleoside bearing a protecting group with a biosynthesis method. Therefore, a series of protective solutions of the present application are proposed.

A first typical implementation of the present application provides a method for enzymatic synthesis of a nucleoside bearing a protecting group. The method uses a purine nucleoside phosphorylase and any one of the following enzymes: a pyrimidine nucleoside phosphorylase or a uracil nucleoside phosphorylase to catalyze a substrate to synthesize the nucleoside bearing the protecting group. The substrate includes a substrate nucleoside, a substrate base, and a substrate phosphate, and the substrate base bears the protecting group; the pyrimidine nucleoside phosphorylase includes PyNP, or a protein that is of more than 80%, 85%, 90%, 95%, 96%, 97%, 98%, 99%, 99.5%, and even 99.9% identity to the PyNP and has the same function, and the PyNP is a protein with the amino acid sequence shown as SEQ ID NO: 1; and the uracil nucleoside phosphorylase includes UP, or a protein that is of more than 80%, 85%, 90%, 95%, 96%, 97%, 98%, 99%, 99.5%, and even 99.9% identity to the UP and has the same function, and the UP is a protein with the amino acid sequence shown as SEQ ID NO: 2. The purine nucleoside phosphorylase includes PNP, or a protein that is of more than 80%, 85%, 90%, 95%, 96%, 97%, 98%, 99%, 99.5%, and even 99.9% identity to the PNP and has the same function, and the PNP includes a protein with the amino acid sequence shown as SEQ ID NO: 3, SEQ ID NO: 7 or SEQ ID NO: 8.

The PNP includes a protein derived from *Geobacillus thermoglucosidasius* and shown as SEQ ID NO: 3, or a protein derived from *Thermus thermophilus* and shown as SEQ ID NO: 7, or a protein derived from *Deinococcus geothermalis* and shown as SEQ ID NO: 8. The above three purine nucleoside phosphorylases all can catalyze the substrate base bearing the protecting group to substitute a phosphate group on a phosphate pentose, so as to obtain the nucleoside bearing the protecting group.

The pyrimidine nucleoside phosphorylase used in the present application is the protein that is derived from the *Thermus thermophilus,* shown as SEQ ID NO: 1, and named as the PyNP; and the uracil nucleoside phosphorylase is a protein that is derived from *Trypanosoma cruzi,* shown as SEQ ID NO: 2, and named as the UP. In the above method, with the purine nucleoside phosphorylase and the pyrimidine nucleoside phosphorylase or the uracil nucleoside phosphorylase, the nucleoside bearing the protecting group can be prepared with the substrate nucleoside and the substrate base bearing the protecting group as raw materials.

The nucleoside bearing the protecting group is a nucleoside bearing a protecting group on a base structure, and the protecting group is the protecting group derived from the substrate base. The protecting group may be a protecting group commonly used in the prior art for protecting a base. The method is a one-step method, can realize one-step preparation in a same container, is easy to perform industrial scale-up production, and can reduce production costs. Furthermore, the method is an enzymatic synthesis method. Compared to a chemical synthesis method, the method is mild and controllable in reaction condition, and not easy to generate other impurities, side-products are less and easy to purify, and a production process is greener, more economical and environmentally friendly.

A reaction route of the method is as follows, and reaction is performed with thymidine as the substrate nucleoside.

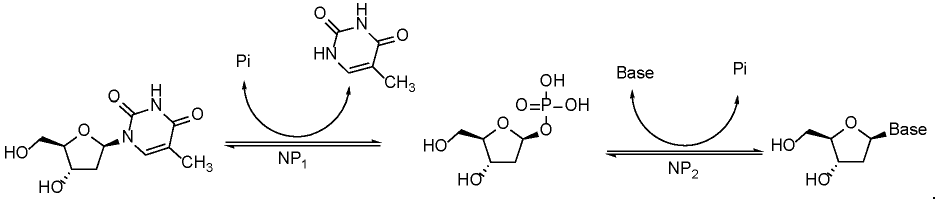

First, a substrate thymidine is catalyzed by a NP₁ enzyme (i.e., the pyrimidine nucleoside phosphorylase or the uracil nucleoside phosphorylase), and a phosphate group (Pi) substitutes a base portion of the substrate nucleoside and is linked onto pentose, so as to form a phosphate pentose and a thymine. Then, under the catalysis of a NP₂ enzyme (i.e., the purine nucleoside phosphorylase), the substrate base substitutes the phosphate group on the phosphate pentose, so as to obtain the nucleoside bearing the protecting group.

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to:
hydrophobic amino acids (Ala, Cys, Gly, Pro, Met, Val, Ile, and Leu) are substituted by other hydrophobic amino acids;
hydrophobic amino acids with bulky side chains (Phe, Tyr, and Trp) are substituted by other hydrophobic amino acids with bulky side chains;
amino acids with positively charged side chains (Arg, His, and Lys) are substituted by other amino acids with positively charged side chains; and
amino acids with polarized and uncharged side chains (Ser, Thr, Asn, and Gln) are substituted by other amino acids with polarized and uncharged side chains.

Those skilled in the art may also make conservative substitutions of the amino acids according to amino acid substitution rules known to those skilled in the art, such as a "blosum62 scoring matrix" in the prior art.

In a preferred embodiment, the protecting group is located on the base structure of the nucleoside. Preferably, the above method includes: when the pyrimidine nucleoside phosphorylase (PyNP) or the uracil nucleoside phosphorylase (UP) is used in combination with the purine nucleoside phosphorylase (PNP), catalyzing the substrate nucleoside and the substrate phosphate with the pyrimidine nucleoside phosphorylase or the uracil nucleoside phosphorylase to generate a phosphate pentose and a free base; and substituting a phosphate group of the phosphate pentose which the substrate base bears the protecting group with the purine nucleoside phosphorylase, so as to obtain the nucleoside bearing the protecting group. The substrate phosphate is not limited to a specific type, and includes, but is not limited to, phosphates commonly used in the prior art, including, but not limited to, one or more of sodium monohydrogen phosphate, sodium dihydrogen phosphate, potassium monohydrogen phosphate, or potassium dihydrogen phosphate, or mixtures such as Phosphate Buffer (PB), Phosphate Buffer Solution (PBS), or the like commercially available in the market. The PB includes a buffer consisting of a certain concentration of the sodium dihydrogen phosphate and disodium hydrogen phosphate. The PBS includes a buffer consisting of the disodium hydrogen phosphate, the potassium dihydrogen phosphate, and salts such as sodium chloride and potassium chloride.

In a preferred embodiment, the substrate nucleoside is a nucleoside shown in a Formula I, where R₁ is selected from -H or -OH or -F, and R₂ is selected from -H or -CH₃; preferably, the substrate nucleoside includes, but is not limited to, thymidine, 2'-deoxyuridine, uridine or 1-beta-D-arabinofuranosyluracil.

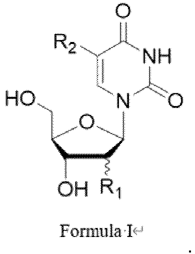

The Formula I is a compound having a chiral site, including a purified chiral compound, and also including a mixture (including, but not limited to, a racemate) bearing different chiral structures. When the R₁ is -OH or -F, the R₁ is a substituent with chirality, in a Haworth projection shown in the Formula I, when the R₁ at a C2 position is in a same direction (the substituent facing downward) with the -OH at an adjacent C3 position, the pentose in the substrate nucleoside is in a ribose configuration, that is, the corresponding substituent is Ribo-OH or Ribo-F; and when the R₁ at the C2 position is in an opposite direction (the substituent facing upward) with the -OH at the adjacent C3 position, the pentose in the substrate nucleoside is in an arabinose configuration, that is, the corresponding substituent is arabino-OH or arabino-F. For the above substrate nucleoside with chirality, the method can prepare the substrate nucleoside into the nucleoside bearing the protecting group without changing a stereochemical configuration of a pentose structure. Furthermore, when the substrate nucleoside is a mixture of different stereochemical configurations, all substrates of different stereochemical configurations can also be converted to obtain the corresponding nucleosides bearing the protecting groups.

In a preferred embodiment, the substrate base is a base shown in a Formula II, a Formula III or a Formula IV, where X, Y and Z are each independently selected from protecting groups commonly used in the prior art such as 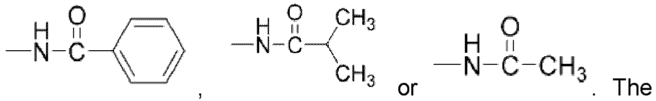 substituent is the protecting group, which can bind to the base structure shown in the Formula II, the Formula III or the Formula IV, so as to protect a binding site.

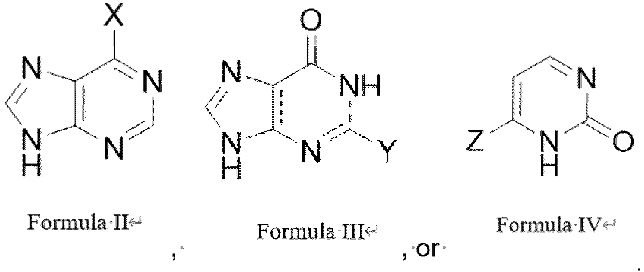

Preferably, the substrate base includes N6-benzoyladenine, N2-isobutyrylguanine or N4-acetylcytosine.

With the substrate nucleoside and the substrate base, the substrate base and the substrate nucleoside can be flexibly combined with the method, so as to prepare the nucleoside bearing the protecting group including but not limited to N6-benzoyladenosine, N2-isobutyrylguanosine, N4-acetylcytidine, N6-benzoyl-2'-deoxyadenosine, N2-isobutyryl-2'-deoxyguanosine, N4-acetyl-2'-deoxycytidine, N6-benzoyl-arabinosyladenosine, or N2-isobutyryl-arabinosylguanosine. The protecting groups are all located on the base structure of the nucleoside, not on the pentose structure.

In a preferred embodiment, one or more of the purine nucleoside phosphorylase, the pyrimidine nucleoside phosphorylase or the uracil nucleoside phosphorylase are purified proteins, crude enzyme liquid or immobilized enzymes.

In a catalytic reaction, the above 3 enzymes may be present in various forms of the purified proteins, the crude enzyme liquid or the immobilized enzymes, and can all be catalyzed and synthesized into the nucleoside bearing the protecting group. A gene expressing the PyNP and/or the UP and/or the PNP is cloned in a host cell, and after protein expression is induced, crude enzyme liquid containing a target protein can be obtained by breaking the host cell. The crude enzyme liquid is simple to prepare, has good catalytic capability, and can reduce the production cost of the catalytic reaction.

In a preferred embodiment, a catalytic time for the enzymatic synthesis is 4-20 h; and preferably, a catalytic temperature of the enzymatic synthesis is 50-70 °C, more preferably, 60 °C.

In a preferred embodiment, a concentration of the substrate nucleoside is 20-400 mM, and a concentration of the substrate base is 10-200 mM.

According to the method, an amplification reaction can be performed, and in the reaction system, the concentration of the substrate nucleoside includes 20, 30, 50, 100, 200, 300, or 400 mM, with a maximum including but not limited to 400 mM. The concentration of the substrate base includes 10, 20, 30, 50, 100, 150, or 200 mM, with a maximum including but not limited to 200 mM, so as to perform large-scale preparation of the nucleoside bearing the protecting group.

Within the above appropriate catalytic temperature and catalytic time, the enzymatic synthesis reaction can be completed, and a conversion rate of the substrate base, i.e., the reaction yield, is higher. Enzymes or other reagents are not required to be added in the middle of the reaction. One-step catalysis can complete the reaction, which is suitable for application in industrial scale-up production. The reaction conditions are mild and easy to control, and the apparatus cost, energy cost, and hazards of production can also be reduced.

In a preferred embodiment, the nucleoside bearing the protecting group includes N6-benzoyladenosine, N2-isobutyrylguanosine, N4-acetylcytidine, N6-benzoyl-2'-deoxyadenosine, N2-isobutyryl-2'-deoxyguanosine, N4-acetyl-2'-deoxycytidine, N6-benzoyl-arabinosyladenosine, or N2-isobutyryl-arabinosylguanosine.

A second typical implementation of the present application provides a composition. The composition includes any one of the following enzymes: a pyrimidine nucleoside phosphorylase or a uracil nucleoside phosphorylase, and a purine nucleoside phosphorylase. The pyrimidine nucleoside phosphorylase includes PyNP, or a protein that is of more than 80%, 85%, 90%, 95%, 96%, 97%, 98%, 99%, 99.5%, and even 99.9% identity to the PyNP and has the same function, and the PyNP is a protein with the amino acid sequence shown as SEQ ID NO: 1; the uridine phosphorylase includes UP, or a protein that is of more than 80%, 85%, 90%, 95%, 96%, 97%, 98%, 99%, 99.5%, and even 99.9% identity to the UP and has the same function, and the UP is a protein with the amino acid sequence shown as SEQ ID NO: 2; and the purine nucleoside phosphorylase includes PNP, or a protein that is of more than 80%, 85%, 90%, 95%, 96%, 97%, 98%, 99%, 99.5%, and even 99.9% identity to the PNP and has the same function, and the PNP includes a protein with the amino acid sequence shown as SEQ ID NO: 3, SEQ ID NO: 7 or SEQ ID NO: 8.

In a preferred embodiment, one or more of the purine nucleoside phosphorylase, the pyrimidine nucleoside phosphorylase or the uracil nucleoside phosphorylase are purified proteins, crude enzyme liquid or immobilized enzymes.

In a preferred embodiment, the composition further includes a substrate nucleoside and a substrate base.

The substrate includes the substrate nucleoside and the substrate base, and the substrate base bears a protecting group.

The substrate nucleoside is a nucleoside shown in a Formula I, where R₁ is selected from -H or -OH or -F, and R₂ is selected from -H or -CH₃.

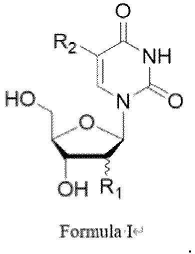

The Formula I is a compound having a chiral site, including a purified chiral compound, and also including a mixture (including, but not limited to, a racemate) containing different chiral structures. When the R₁ is -OH or -F, the R₁ is a substituent with chirality, in a Haworth projection shown in the Formula I, when the R₁ at a C2 position is in a same direction (the substituent facing downward) with the -OH at an adjacent C3 position, the pentose in the substrate nucleoside is in a ribose configuration, that is, the corresponding substituent is Ribo-OH or Ribo-F; and when the R₁ at the C2 position is in an opposite direction (the substituent facing upward) with the -OH at the adjacent C3 position, the pentose in the substrate nucleoside is in an arabinose configuration, that is, the corresponding substituent is arabino-OH or arabino-F. The composition includes a purified chiral compound, and also includes a mixture of compounds with different chirality, including, but not limited to, a racemate. Preferably, the substrate nucleoside includes thymidine, 2'-deoxyuridine, uridine or 1-beta-D-arabinofuranosyluracil.

Preferably, the substrate base is a base shown in a Formula II, a Formula III or a Formula IV, where X, Y and Z are each independently selected from

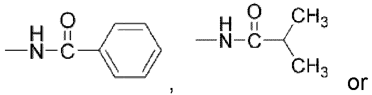

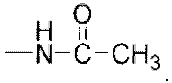

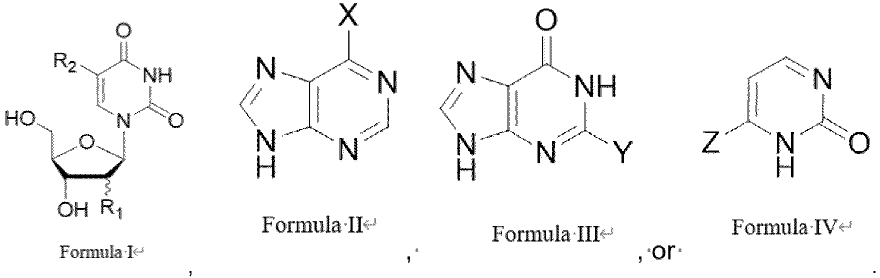

The substituent is the protecting group, which can bind to the base structure shown in the Formula II, the Formula III or the Formula IV, so as to protect a binding site.

Preferably, the substrate base includes N6-benzoyladenine, N2-isobutyrylguanine or N4-acetylcytosine.

Preferably, a concentration of the substrate nucleoside is 20-400 mM, and a concentration of the substrate base is 10-200 mM.

The concentration of the substrate nucleoside includes 20, 30, 50, 100, 200, 300, or 400 mM, with a maximum including but not limited to 400 mM. The concentration of the substrate base includes 10, 20, 30, 50, 100, 150, or 200 mM, with a maximum including but not limited to 200 mM.

The pyrimidine nucleoside phosphorylase PyNP is a protein that is derived from *Thermus thermophilus* and shown as SEQ ID NO: 1. The uracil nucleoside phosphorylase UP is a protein that is derived from *Trypanosoma cruzi* and shown as SEQ ID NO: 2. The purine nucleoside phosphorylase PNP includes a protein that is shown as SEQ ID NO: 3 and derived from *Geobacillus thermoglucosidasius,* or a protein that is shown as SEQ ID NO: 7 and derived from *Thermus thermophilus,* or a protein that is shown as SEQ ID NO: 8 and derived from *Deinococcus geothermalis.* The composition can catalyze the substrate base and the substrate nucleoside to undergo a reaction, so as to generate the nucleoside bearing the protecting group. The enzymes in the composition can be each independently selected from forms such as purified proteins, crude enzyme liquid or immobilized enzymes, and can all achieve a catalysis effect.

The beneficial effects of the present application are further described in detail below with reference to specific embodiments.

### Embodiment 1

### 1. Strain construction

A used pyrimidine nucleoside phosphorylase PyNP was derived from *Thermus thermophilus; a* uracil nucleoside phosphorylase UP was derived from *Trypanosoma cruzi;* and a purine nucleoside phosphorylase PNP was derived from *Geobacillus thermoglucosidasius.* Sequences of proteins of the three nucleoside phosphorylases obtained through NCBI were respectively SEQ ID NO: 1, SEQ ID NO: 2 and SEQ ID NO: 3 (NCBI protein serial number: EFG53380.1). DNA sequences encoding three enzymes obtained after codon optimization were respectively SEQ ID NO: 4, SEQ ID NO: 5, and SEQ ID NO: 6, and were cloned onto an expression vector pET28a (+). Obtained plasmids were transferred into *Escherichia coli* BL21 (DE3) host competence, so as to obtain monoclonal strains.
SEQ ID NO: 1: PyNP: 
MNPVAFIREKREGKKHRREDLEAFLLGYLRDEVPDYQVSAWLMAAFLRGLDPEETLWLTETM
ARSGKVLDLSGLPHPVDKHSSGGVGDKVSLVVGPILAASGCTFAKMSGRGLAHTGGTIDKLESVP
GWRGEMTEAEFLERARRVGLVIAAQSPDLAPLDGKLYALRDVTATVESVPLIASSIMSKKLAAGARS
IVLDVKVGRGAFMKTLEEARLLAKTMVAIGQGAGRRVRALLTSMEAPLGRAVGNAIEVREAIEALKG
EGPGDLLEVALALAEEALRLEGLDPALARKALEGGAALEKFRAFLEAQGGDPRAVEDFSLLPLAEE
HPLRAEREGVVREVDAYKVGLAVLALGGGRKRKGEPIDHGVGVYLLKKPGDRVERGEALALVYHR
RRGLEEALGHLREAYALGEEAHPAPLVLEAI.
SEQ ID NO: 2: UP: 
MSKATHTHGLMKDPDLPVTADGVTYHLNCKSDQLADRILLVGDPGRVDTVAACFDAGSVRFR
SENREIFIATGTYKGTPVSVLSTGMGTDNVEIVINEIHLLKEYDFERCSWRPRVGDADVPPGAKVFD
PSSVKMIRVGTCGSPFPDMPLAALAITRHVIGMDNTCLYYKVKEQMQKSKDLQEVQRVVKEQTSL
GAIDVYATKAHPAITNGIVAACKAFNDNLPAGATEQLYVVGATATASGFYACQGRAVGRFREHLLVP
KLVEELSKVQFDVSEGKEIVANIEMETSAVCFLSHLLGYQAGSMCVVVAKRAGAEHLFTTSEQSGQ
ALKNAIQIALEAIVAVG.
SEQ ID NO: 3: PNP: 
MSIHIEAKQQEIAEKILLPGDPLRAQYIAETFLEGATCYNRVRGMLGFTGTYKGHRISVQGTGM
GVPSISIYVNELIQSYHVQTLIRVGTCGAIQKDVNVRDVILAMSASTDSNMNRLTFRGRDYAPTANF
ALLRTAYEVGAEKGLPLKVGSVFTADMFYNDEPDWETWARYGVLAVEMETAALYTLAAKFGRKAL
SVLTVSDHILTGEETTAQERQTTFNDMIEVALETAIRVE.
SEQ ID NO: 4: 
atgaacccggtggcgtttattcgcgaaaaacgcgaaggcaagaaacatcgccgcgaggatctggaagcgtttctgctgggctatct
gcgcgatgaagtgccggattatcaggtgagcgcgtggctgatggcggcgtttttacgcggcttagatccggaagaaaccctgtggctgac
cgaaacgatggcccgcagcggtaaagtgctggatctgagcggtctgccgcatccggttgataaacatagcagcggcggcgtgggcgat
aaagtgagcttagtggtgggcccgattctggcggcgagcggttgtacctttgcgaaaatgtcgggccgcggcctggcgcataccggtggt
accattgataaactggaaagcgtgccgggctggcgcggcgaaatgaccgaagcagaatttctggaacgcgcccgtcgcgttggtctggtt
attgcggcgcaaagcccggatttagcgccgttagatggcaaactgtatgcgctgcgcgatgtgaccgcgaccgttgaatcagttccgctga
ttgcgagcagcattatgagcaagaaactggcggcgggcgcccgtagcattgtgttagatgttaaagtgggccgcggcgcgtttatgaaaa
ccctggaagaagcgcgtctgctggcgaaaacgatggtggcgattggccagggcgcgggccgtcgtgttcgtgcgttattaaccagcatgg
aagcgcctctgggtcgtgcggttggcaatgcgattgaagtgcgtgaagcgattgaagcgctgaaaggcgaaggcccgggcgatttatta
gaagtggcgctggcgttagcggaagaagcgctgcgtttagaaggcttagatccggcgctggcgcgtaaagcgttagaaggtggcgcgg
cgctggaaaaatttcgcgcgtttctggaagcgcagggcggcgatccgcgtgcagttgaagattttagcctgctgccgctggcggaagaac
atccgttacgtgcggaacgcgaaggcgtggttcgcgaagttgatgcgtataaagtgggcctggcggtgctggcgttaggcggtggtcgta
aacgtaaaggtgaaccgattgatcatggcgtgggcgtgtatctgctgaagaaaccgggcgatcgcgtggaacgcggcgaagcgttagc
gttagtgtatcatcgccgccgcggcttagaagaagcgctgggtcatttacgcgaagcgtatgcgctgggcgaagaagcgcatccggcgc
ctctggttttagaagcgatttaa.
SEQ ID NO: 5: 
atgagcaaagcgacccatacccacggcctgatgaaagatccggatctgccggtgacggcggatggcgttacctatcatctgaact
gcaaaagcgatcagctggcggatcgcattctgctggtgggcgatccgggtcgcgttgataccgtagcggcatgttttgatgcgggcagcgt
tcgctttcgcagcgaaaaccgcgaaatttttattgcgaccggcacctataaaggcaccccggtgtcagtgctgagcaccggtatgggtact
gataatgtggaaattgtgattaacgagattcacctgctgaaggagtatgacttcgagcgctgcagctggcgcccgcgtgttggtgatgcgg
atgttcctcctggtgcgaaagtgtttgatccgagcagcgtgaaaatgattcgcgtgggcacctgcggcagcccgtttcctgatatgcctctgg
cggcgctggcgattacccgccatgttattggcatggataacacctgcctgtattataaagtgaaagagcagatgcagaagagcaaggatc
tgcaggaagtgcagcgcgtggtgaaagaacagaccagcctgggcgcgattgatgtgtatgcgaccaaagcgcatccggcgattacca
acggcattgtggcggcgtgtaaagcgtttaacgataacctgccggcgggcgcgactgaacaattatatgttgttggcgcgaccgcgaccg
cgagcggtttttatgcgtgtcaaggtcgcgcggtgggccgctttcgtgaacatttactggttccgaaactggtggaagaactgagcaaagtg
cagtttgatgtgagcgaaggcaaagaaattgtggcgaacattgaaatggaaaccagcgcggtgtgctttctgagccatctgctgggctatc
aggcgggctcaatgtgcgttgttgttgcgaaacgcgcgggcgcggaacatctgtttaccaccagcgaacagagcggccaggcgctgaa
aaacgcgattcagattgcgctggaagcgattgtggcggtgggctaa.
SEQ ID NO: 6:

atgagcattcatattgaagcgaaacagcaggaaattgcggaaaagattctgctgccgggcgatccgttacgtgcgcaatatattgc
ggaaacctttctggaaggcgcgacctgctataaccgcgtgcgcggtatgctgggctttaccggtacctataaaggccatcgcattagcgtg
cagggcaccggcatgggcgttccgtcaattagcatttatgtgaacgaactgattcagagctatcacgtgcagaccctgattcgcgtgggca
cctgtggcgcaattcagaaagatgtgaacgtgcgcgatgtgattctggcgatgagcgcgagcaccgatagcaacatgaaccgcctgacc
tttcgcggccgtgattatgcgcctaccgcgaattttgcgctgctgcgcactgcgtatgaagtgggcgcggaaaaaggcctgccgctgaaag
tgggcagcgtgtttaccgcggatatgttttataacgatgaaccggattgggaaacctgggcccgctatggcgttctggcggtggaaatgga
aaccgcggcgttatataccctggcggcgaaatttggccgcaaagcgctgagcgtgctgaccgtgagcgatcatattctgaccggcgaag
aaaccaccgcgcaggaacgccaaaccacctttaacgatatgattgaagtggcgctggaaaccgcgattcgcgtggaataa.

### 2. Protein expression

The *Escherichia coli* strains expressing the PyNP, the UP, and the PNP were respectively inoculated into test tubes, cultured at 37 °C for 16 h, and then inoculated into 2L shake flasks containing 500 mL of Luria-Bertani(LB) according to a 1% inoculation amount; culture was performed at 37 °C until OD₆₀₀ was 0.6, Isopropyl β-D-1-thiogalactopyranoside (IPTG)with a final concentration being 0.1 M was added to induce protein expression; and culture was performed at 20 °C for 18 h. The cultured bacterial fluid was centrifuged at 7000 rpm for 10 min, and then bacterial cells were collected for later use.

### 3. Enzyme liquid preparation

0.1 g of bacterial sludge was weighed, 1 mL of a potassium phosphate buffer with pH being 7.5 was added, after shaking and well mixing, a sonicator was used to crush bacterial suspension for 5 min with power of 30%. A catalytic reaction was performed with the enzyme liquid prepared by the above three proteins in Embodiments 2-13.

### 4. HPLC detection method

A chromatographic column used an Atlantis T3 Column of 4.6 mm x 150 mm, a mobile phase was water/acetonitrile with a flow rate being 1 mL/min and a temperature being 40 °C, and an UV detector was used with a detection wavelength being 254 nm and a detection duration being 15 min.

### Embodiment 2 Synthesis of N6-benzoyladenosine using uridine as substrate

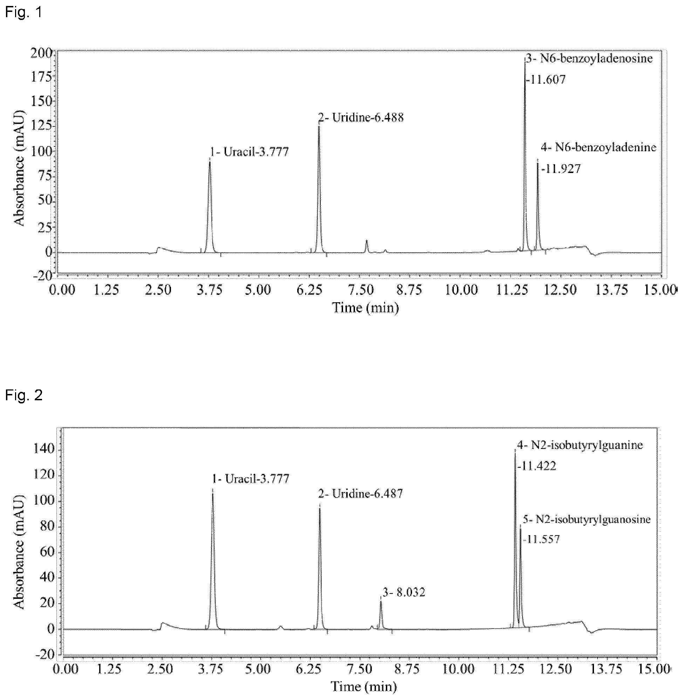

In a reaction system containing 2 mM phosphate buffer (PB, pH being 7.5), 20 mM uridine (final concentration), 10 mM N6-benzoyladenine (final concentration), 4.88 mg of PyNP enzyme liquid prepared by bacterial sludge, and 2.39 mg of PNP enzyme liquid prepared by the bacterial sludge were added, with a total reaction volume of 1 mL. And after the reaction was performed at 60 °C for 4 h, an equal volume of Dimethyl Sulfoxide (DMSO) was added, and HPLC detection was performed after dilution by a certain multiple, as shown in Fig. 1. Results showed that a conversion rate of the uridine was 51.03% (i.e., 51.03% of the uridine was converted into uracil and phosphate pentose), and a conversion rate of the N6-benzoyladenine was 68.20% (i.e., 68.20% of the N6-benzoyladenine bound to the phosphate pentose to generate the N6-benzoyladenosine).

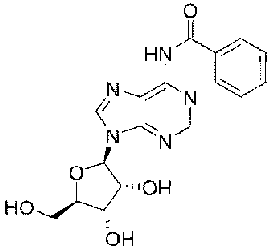

### N6-Benzoyladenosine

### Embodiment 3 Synthesis of N2-isobutyrylguanosine using uridine as substrate

In a reaction system containing 2 mM phosphate buffer (pH being 7.5), 20 mM uridine (final concentration), 10 mM N2-isobutyrylguanine (final concentration), 4.88 mg of PyNP enzyme liquid prepared by bacterial sludge, and 2.21 mg of PNP enzyme liquid prepared by the bacterial sludge were added, with a total reaction volume of 1 mL. And after the reaction was performed at 60 °C for 19 h, an equal volume of DMSO was added, and HPLC detection was performed after dilution by a certain multiple, as shown in Fig. 2. Results showed that a conversion rate of the uridine was 62.16%, and a conversion rate of the N2-isobutyrylguanine was 36.51%.

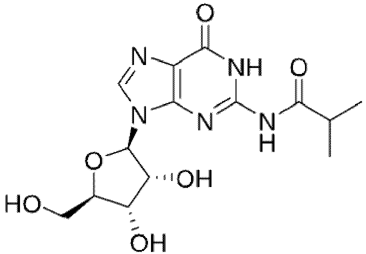

### N2-Isobutyrylguanosine

### Embodiment 4 Synthesis of N4-acetylcytidine using uridine as substrate

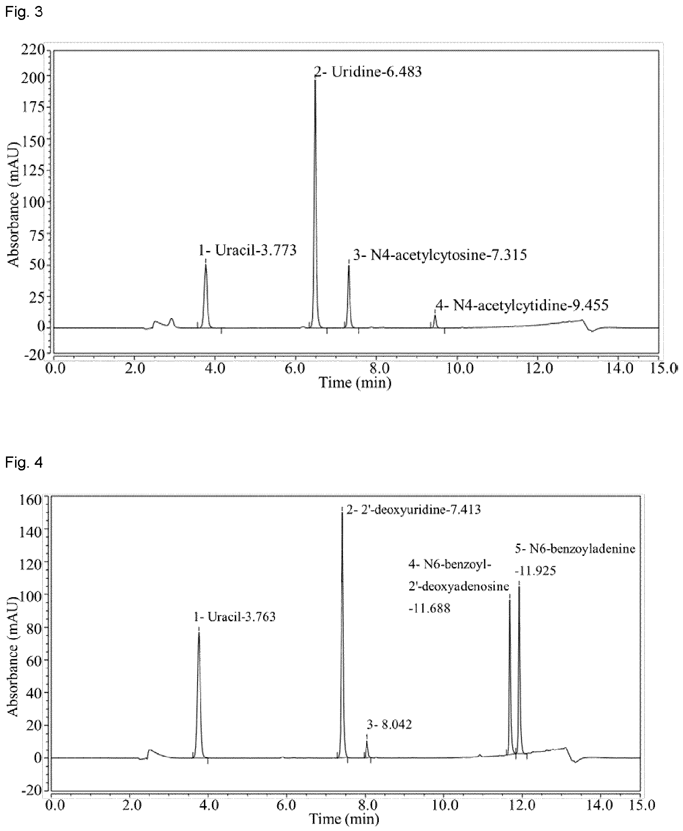

In a reaction system containing 2 mM phosphate buffer (pH being 7.0), 20 mM uridine (final concentration), 10 mM N4-acetylcytosine (final concentration), 4.88 mg of PyNP enzyme liquid prepared by bacterial sludge, and 1.53 mg of PNP enzyme liquid prepared by the bacterial sludge were added, with a total reaction volume of 1 mL. And after the reaction was performed at 60 °C for 18 h, an equal volume of DMSO was added, and HPLC detection was performed after dilution by a certain multiple, as shown in Fig. 3. Results showed that a conversion rate of the uridine was 27.35%, and a conversion rate of the N4-acetylcytosine was 15.67%.

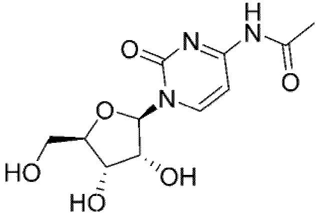

### N4-Acetylcytidine

### Embodiment 5 Synthesis of N6-benzoyl-2'-deoxyadenosine using 2'-deoxyuridine as substrate

In a reaction system containing 2 mM phosphate buffer (pH being 7.5), 20 mM 2'-deoxyuridine (final concentration), 10 mM N6-benzoyladenine (final concentration), 4.56 mg of PyNP enzyme liquid prepared by bacterial sludge, and 2.39 mg of PNP enzyme liquid prepared by the bacterial sludge were added, with a total reaction volume of 1 mL. And after the reaction was performed at 60 °C for 4 h, an equal volume of DMSO was added, and HPLC detection was performed after dilution by a certain multiple, as shown in Fig. 4. Results showed that a conversion rate of the 2'-deoxyuridine was 45.23%, and a conversion rate of the N6-benzoyladenine was 47.83%.

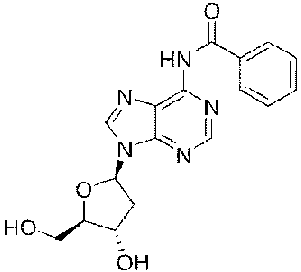

### N6-Benzoyl-2'-deoxyadenosine

### Embodiment 6 Synthesis of N2-isobutyryl-2'-deoxyguanosine using 2'-deoxyuridine as substrate

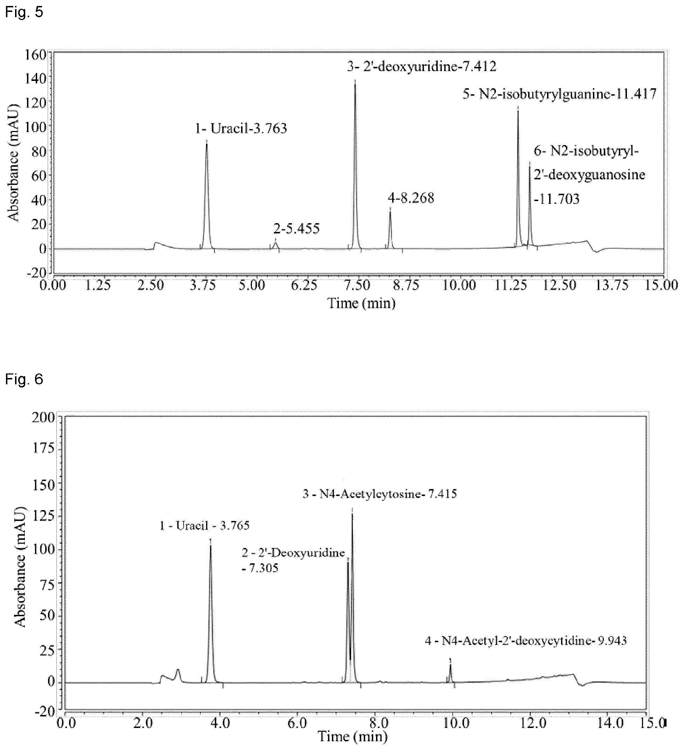

In a reaction system containing 2 mM phosphate buffer (pH being 7.5), 20 mM 2'-deoxyuridine (final concentration), 10 mM N2-isobutyrylguanine (final concentration), 4.56 mg of PyNP enzyme liquid prepared by bacterial sludge, and 2.21 mg of PNP enzyme liquid prepared by the bacterial sludge were added, with a total reaction volume of 1 mL. And after the reaction was performed at 60 °C for 19 h, an equal volume of DMSO was added, and HPLC detection was performed after dilution by a certain multiple, as shown in Fig. 5. Results showed that a conversion rate of the 2'-deoxyuridine was 50.59%, and a conversion rate of the N2-isobutyrylguanine was 35.52%.

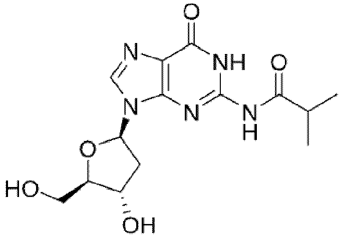

### N2-Isobutyryl-2'-deoxyguanosine

### Embodiment 7 Synthesis of N4-acetyl-2'-deoxycytidine using 2'-deoxyuridine as substrate

In a reaction system containing 2 mM phosphate buffer (pH being 7.0), 20 mM 2'-deoxyuridine (final concentration), 10 mM N4-acetylcytosine (final concentration), 4.56 mg of PyNP enzyme liquid prepared by bacterial sludge, and 1.53 mg of PNP enzyme liquid prepared by the bacterial sludge were added, with a total reaction volume of 1 mL. And after the reaction was performed at 60 °C for 18 h, an equal volume of DMSO was added, and HPLC detection was performed after dilution by a certain multiple, as shown in Fig. 6. Results showed that a conversion rate of the 2'-deoxyuridine was 56.21%, and a conversion rate of the N4-acetylcytosine was 9.71%.

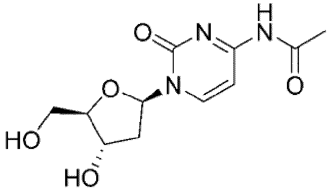

### N4-Acetyl-2'-deoxycytidine

### Embodiment 8 Synthesis of N6-benzoyl-2'-deoxyadenosine using thymidine as substrate

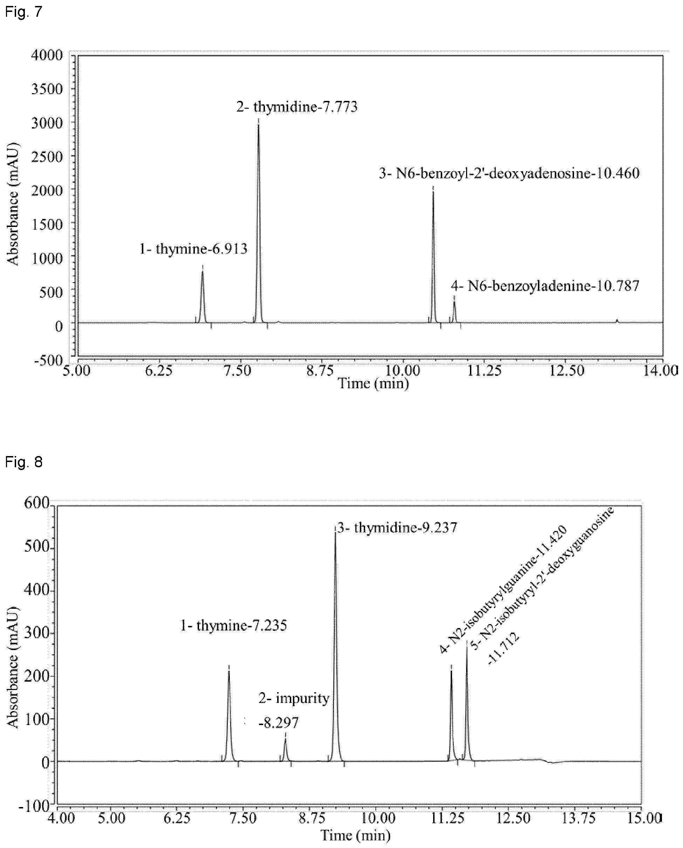

In a reaction system containing 2 mM phosphate buffer (pH being 7.5), 40 mM thymidine (final concentration), 10 mM N6-benzoyladenine (final concentration), 0.97 mg of PyNP enzyme liquid prepared by bacterial sludge, 0.24 mg of PNP enzyme liquid prepared by the bacterial sludge and 20% of N,N-dimethylformamide (DMF) (final volume percentage) were added, with a total reaction volume of 1 mL. And after the reaction was performed at 60 °C for 4 h, an equal volume of DMSO was added, HPLC detection was performed after dilution by a certain multiple, and 0.1% of Trifluoroacetic Acid (TFA) was added in a mobile phase, as shown in Fig. 7. Results showed that a conversion rate of the thymidine was 22.08%, and a conversion rate of the N6-benzoyladenine was 85.7%.

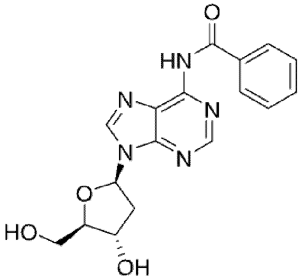

### N6-Benzoyl-2'-deoxyadenosine

### Embodiment 9 Synthesis of N2-isobutyryl-2'-deoxyguanosine using thymidine as substrate

In a reaction system containing 2 mM phosphate buffer (pH being 7.5), 60 mM thymidine (final concentration), 20 mM N2-isobutyrylguanine (final concentration), 13.57 mg of PyNP enzyme liquid prepared by bacterial sludge, 35.33 mg of PNP enzyme liquid prepared by the bacterial sludge and 15% of DMSO (final volume percentage) were added, with a total reaction volume of 1 mL. And after the reaction was performed at 60 °C for 19 h, an equal volume of the DMSO was added, and HPLC detection was performed after dilution by a certain multiple, as shown in Fig. 8. Results showed that a conversion rate of the thymidine was 29.98%, and a conversion rate of the N2-isobutyrylguanine was 62.49%.

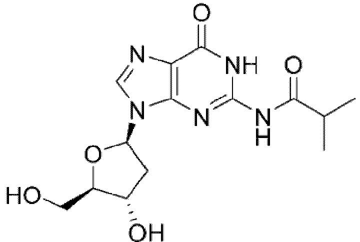

### N2-Isobutyryl-2'-deoxyguanosine

### Embodiment 10 Amplification reaction of synthesizing N6-benzoyl-2'-deoxyadenosine using thymidine as substrate

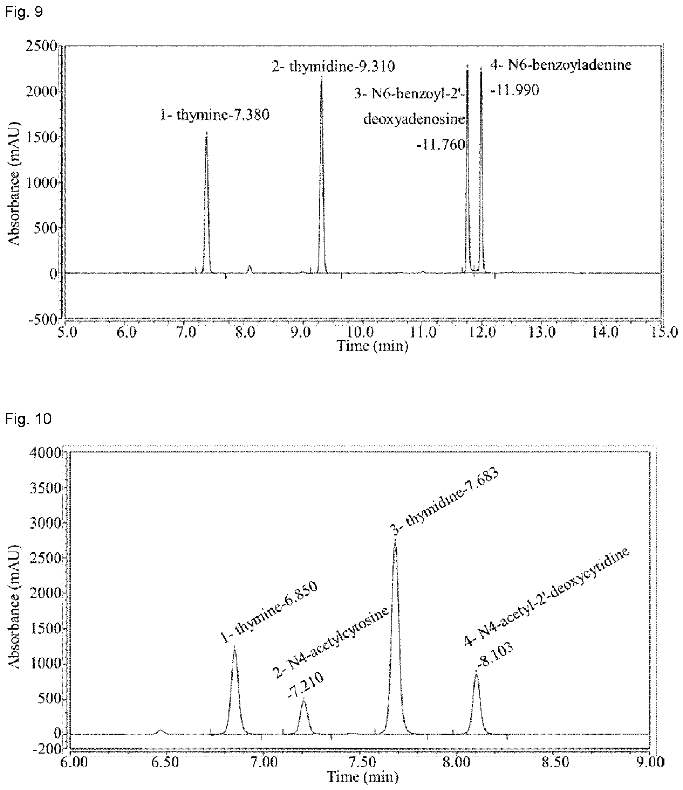

In a reaction system containing 2 mM phosphate buffer (pH being 7.5), 400 mM thymidine (final concentration), 200 mM N6-benzoyladenine (final concentration), 9.68 g of PyNP enzyme liquid prepared by bacterial sludge, and 4.78 g of PNP enzyme liquid prepared by the bacterial sludge were added, with a total reaction volume of 100 mL. And after the reaction was performed at 60 °C for 16 h, an equal volume of DMSO was added, and HPLC detection was performed after dilution by a certain multiple, as shown in Fig. 9. In the amplification reaction, compared to a small trial, a higher product yield could be obtained by increasing a proportion of a substrate base. Results showed that a conversion rate of the thymidine was 43.12%, and a conversion rate of the N6-benzoyladenine was 50.05%.

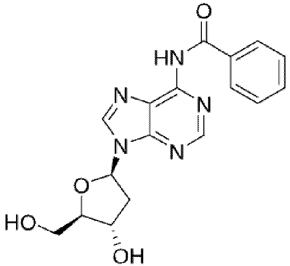

### N6-Benzoyl-2'-deoxyadenosine

### Embodiment 11 Reaction of synthesizing N4-acetyl-2'-deoxycytidine using thymidine as substrate

In a reaction system containing 2 mM phosphate buffer (pH being 7.0), 400 mM thymidine (final concentration), 100 mM N4-acetylcytosine (final concentration), 9.04 mg of PyNP enzyme liquid prepared by bacterial sludge, and 76.56 mg of PNP enzyme liquid prepared by the bacterial sludge were added, with a total reaction volume of 1 mL. And after the reaction was performed at 60 °C for 18 h, an equal volume of DMSO was added, and HPLC detection was performed after dilution by a certain multiple, as shown in Fig. 10. Results showed that a conversion rate of the thymidine was 30.52%, and a conversion rate of the N4-acetylcytosine was 63.70%.

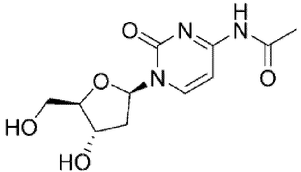

### N4-Acetyl-2'-deoxycytidine

### Embodiment 12 Synthesis of N6-benzoyl-arabinosyladenosine using 1-beta-D-arabinofuranosyluracil as substrate

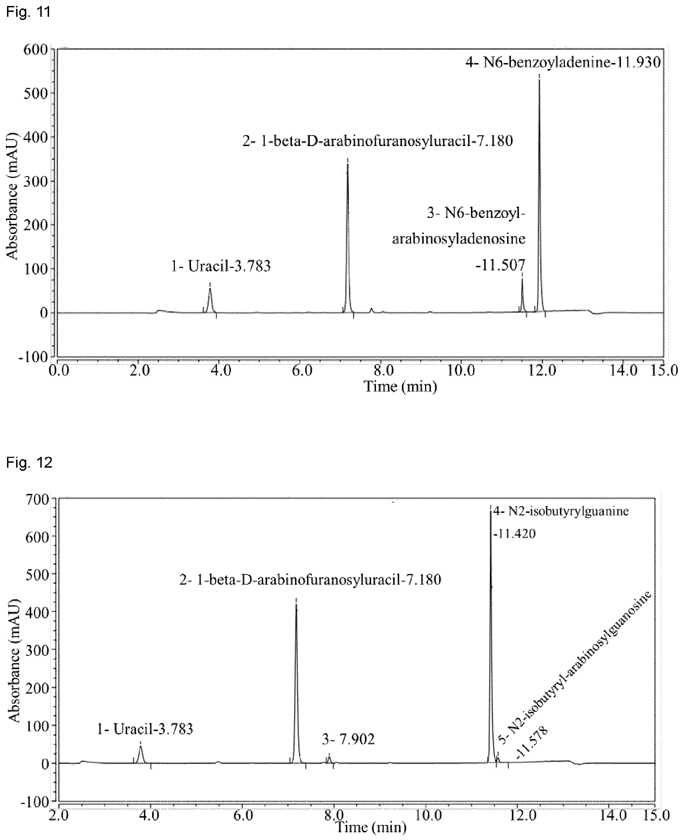

In a reaction system containing 2 mM phosphate buffer (pH being 7.5), 40 mM 1-beta-D-arabinofuranosyluracil (final concentration), 20 mM N6-benzoyladenine (final concentration), 9.76 mg of UP enzyme liquid prepared by bacterial sludge, and 4.78 mg of PNP enzyme liquid prepared by the bacterial sludge were added, with a total reaction volume of 1 mL. And after the reaction was performed at 60 °C for 16 h, an equal volume of Dimethyl Sulfoxide (DMSO) was added, and HPLC detection was performed after dilution by a certain multiple, as shown in Fig. 11. Results showed that a conversion rate of the 1-beta-D-arabinofuranosyluracil was 20.73%, and a conversion rate of the N6-benzoyladenine was 12.24%.

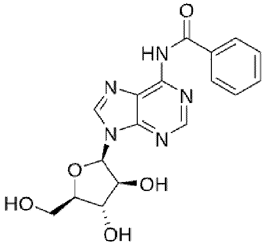

### N6-Benzoyl-arabinosyladenosine

### Embodiment 13 Synthesis of N2-isobutyryl-arabinosylguanosine using 1-beta-D-arabinofuranosyluracil as substrate

In a reaction system containing 2 mM phosphate buffer (pH being 7.5), 40 mM 1-beta-D-arabinofuranosyluracil (final concentration), 20 mM N2-isobutyrylguanine (final concentration), 9.76 mg of UP enzyme liquid prepared by bacterial sludge, and 4.42 mg of PNP enzyme liquid prepared by the bacterial sludge were added, with a total reaction volume of 1 mL. And after the reaction was performed at 60 °C for 16 h, an equal volume of DMSO was added, and HPLC detection was performed after dilution by a certain multiple, as shown in Fig. 12. Results showed that a conversion rate of the 1-Beta-D-arabinofuranosyluracil was 14.87%, and a conversion rate of the N2-isobutyrylguanine was 2.00%.

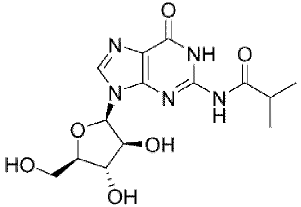

### N2-Isobutyryl-arabinosylguanosine

### Embodiment 14 Synthesis of N6-benzoyl-2'-deoxyadenosine with participation of other sources of PNPs

In a reaction system containing 2 mM phosphate buffer (pH being 7.5), 400 mM thymidine (final concentration), 200 mM N6-benzoyladenine (final concentration), 9.68 g of PyNP enzyme liquid prepared by bacterial sludge, and 4.78 g of PNP enzyme liquid prepared by the bacterial sludge were added, with a total reaction volume of 1 mL. After the reaction was performed at 60 °C for 16 h, an equal volume of DMSO was added, and HPLC detection was performed after dilution by a certain multiple; and the PNPs were respectively derived from *Thermus thermophilus* and *Deinococcus geothermalis.* A sequence of a protein of the PNP derived from the *Thermus thermophilus* was SEQ ID NO: 7, and a conversion rate of the corresponding N6-benzoyladenine was 48.79%. A sequence of a protein of the PNP derived from the *Deinococcus geothermalis* was SEQ ID NO: 8, and a conversion rate of the corresponding N6-benzoyladenine was 46.51%, which was close to the conversion rate 48.84% of the PNP derived from *Geobacillus thermoglucosidasius.*
SEQ ID NO: 7: 
MPGMELYDKIQEAVAYVRSKTDFVPEVGLVLGSGLGPLADEVEKVAEIPYGEIPHFPVSTAPGH
AGRLVLGRLEGKPVLVYKGRVHYYEGYSAEEVVFPVRVGFFLGARTFLLTSAAGGLNPRFRAGGI
MLHLDYINFAGANPLRGPNDERLGPRFPVMFEAYDPELIELARKVARRQDLHLFEGVYAWFMGPS
FASRAELKLLRELGADAIGMSTVPEVIALRHLGARVLGLSTITDMAVPEREHHATEEEVLRVAAETG
 PVFRRYVRGILAEL.
SEQ ID NO: 8:

MVARVPARPFASPPATLDRVSVHLNARPGEIAETVLLPGDPLRAQHIAETFFENPVQHNSVRG
MLGFTGTYRGVPVSVQGTGMGIASSMIYVNELIRDYGCQTLIRVGTAGSYQPDVHVRDLVLAQAA
CTDSNINNIRFGLRNFAPIADFELLLRAYQMARDRGFATHVGNILSSDTFYQDDPESYKLWAQYGVL
AVEMEAAGLYTLAAKYGVRALTILTISDHLVTREETTAEERQTTFNGMIEVALDAALGLAVPSNSM.

From the above descriptions, it might be seen that the embodiments of the present application achieved the following technical effects. With the purine nucleoside phosphorylase and the pyrimidine nucleoside phosphorylase or the uracil nucleoside phosphorylase, enzymatic catalysis could be performed with the substrate nucleoside and the substrate base bearing the protecting group, so as to synthesize the nucleoside bearing the protecting group with a biosynthesis method; and scale-up production could be performed, so as to prepare large amount of the nucleosides bearing the protecting group within a short time and under mild reaction conditions.

The above are only the preferred embodiments of the present application and are not intended to limit the present application. For those skilled in the art, the present application may have various modifications and variations. Any modifications, equivalent replacements, improvements and the like made within the spirit and principle of the present application all fall within the scope of protection of the present application.

## Claims

1. A method for enzymatic synthesis of a nucleoside bearing a protecting group, comprising:
using a purine nucleoside phosphorylase and any one of following: a pyrimidine nucleoside phosphorylase or a uridine phosphorylase, to catalyze a substrate so as to synthesize a nucleoside bearing a protecting group, wherein
the substrate comprises a substrate nucleoside, a substrate base, and a substrate phosphate, and the substrate base bears the protecting group;
the pyrimidine nucleoside phosphorylase comprises PyNP, or a protein that is of more than 80% identity to the PyNP and has the same function, and the PyNP is a protein with the amino acid sequence shown as SEQ ID NO: 1;
the uridine phosphorylase comprises UP, or a protein that is of more than 80% identity to the UP and has the same function, and the UP is a protein with the amino acid sequence shown as SEQ ID NO: 2; and
the purine nucleoside phosphorylase comprises PNP, or a protein that is of more than 80% identity to the PNP and has the same function, and the PNP comprises a protein with the amino acid sequence shown as SEQ ID NO: 3, SEQ ID NO: 7 or SEQ ID NO: 8.

2. The method according to claim 1, wherein the protecting group is located on a base structure of the nucleoside;
preferably, the method comprises:
catalyzing the substrate nucleoside and the substrate phosphate with the pyrimidine nucleoside phosphorylase or the uracil nucleoside phosphorylase to generate a phosphate pentose and a free base; and
substituting a phosphate group of the phosphate pentose which the substrate base bears the protecting group with the purine nucleoside phosphorylase, so as to obtain the nucleoside bearing the protecting group.

3. The method according to claim 1, wherein the substrate nucleoside is a nucleoside shown in a Formula I, wherein R₁ is selected from -H or -OH or -F, and R₂ is selected from -H or -CH₃; and 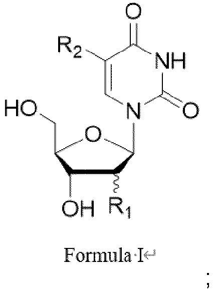 preferably, the substrate nucleoside comprises thymidine, 2'-deoxyuridine, uridine or 1-beta-D-arabinofuranosyluracil.

4. The method according to claim 1, wherein the substrate base is a base shown in a Formula II, a Formula III or a Formula IV, wherein X, Y and Z are each independently selected from 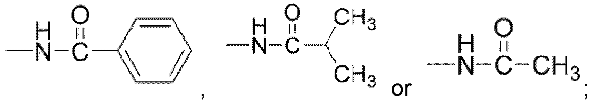 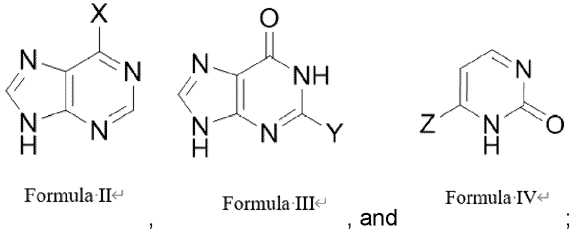
preferably, the substrate base comprises N6-benzoyladenine, N2-isobutyrylguanine or N4-acetylcytosine; and
preferably, the substrate phosphate comprises one or more of sodium monohydrogen phosphate, sodium dihydrogen phosphate, potassium monohydrogen phosphate, or potassium dihydrogen phosphate.

5. The method according to claim 1, wherein one or more of the purine nucleoside phosphorylase, the pyrimidine nucleoside phosphorylase or the uracil nucleoside phosphorylase are purified proteins, crude enzyme liquid or immobilized enzymes.

6. The method according to claim 1, wherein a catalytic time for the enzymatic synthesis is 4-20 h;
preferably, a catalytic temperature of the enzymatic synthesis is 50-70 °C, more preferably, 60 °C; and
preferably, a concentration of the substrate nucleoside is 20-400 mM, a concentration of the substrate base is 10-200 mM, and a concentration of the substrate phosphate is 1-100 mM.

7. The method according to claim 1, wherein the nucleoside bearing the protecting group comprises N6-benzoyladenosine, N2-isobutyrylguanosine, N4-acetylcytidine, N6-benzoyl-2'-deoxyadenosine, N2-isobutyryl-2'-deoxyguanosine, N4-acetyl-2'-deoxycytidine, N6-benzoyl-arabinosyladenosine, or N2-isobutyryl-arabinosylguanosine.

8. A composition, comprising any one of the following enzymes: a pyrimidine nucleoside phosphorylase or a uracil nucleoside phosphorylase, and a purine nucleoside phosphorylase, wherein
the pyrimidine nucleoside phosphorylase comprises PyNP, or a protein that is of more than 80% identity to the PyNP and has the same function, and the PyNP is a protein with the amino acid sequence shown as SEQ ID NO: 1;
the uridine phosphorylase comprises UP, or a protein that is of more than 80% identity to the UP and has the same function, and the UP is a protein with the amino acid sequence shown as SEQ ID NO: 2; and
the purine nucleoside phosphorylase comprises PNP, or a protein that is of more than 80% identity to the PNP and has the same function, and the PNP comprises a protein with the amino acid sequence shown as SEQ ID NO: 3, SEQ ID NO: 7 or SEQ ID NO: 8.

9. The composition according to claim 8, wherein one or more of the purine nucleoside phosphorylase, the pyrimidine nucleoside phosphorylase or the uracil nucleoside phosphorylase are purified proteins, crude enzyme liquid or immobilized enzymes.

10. The composition according to claim 8 or 9, further comprising a substrate nucleoside, a substrate base, and a substrate phosphate, wherein
the substrate base bears a protecting group;
the substrate nucleoside is a nucleoside shown in a Formula I, wherein R₁ is selected from -H or -OH or -F, and R₂ is selected from -H or -CH₃; 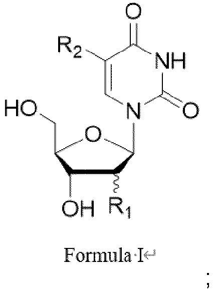
preferably, the substrate nucleoside comprises thymidine, 2'-deoxyuridine, uridine or 1-beta-D-arabinofuranosyluracil;
preferably, the substrate base is a base shown in a Formula II, a Formula III or a Formula IV,
wherein X, Y and Z are each independently selected from 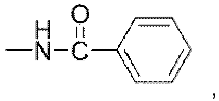 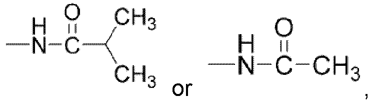 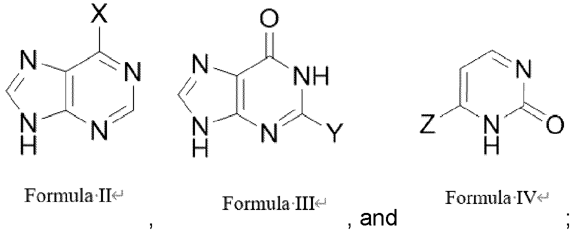
preferably, the substrate base comprises N6-benzoyladenine, N2-isobutyrylguanine or N4-acetylcytosine; and
preferably, the substrate phosphate comprises one or more of sodium monohydrogen phosphate, sodium dihydrogen phosphate, potassium monohydrogen phosphate, or potassium dihydrogen phosphate; and
preferably, a concentration of the substrate nucleoside is 20-400 mM, a concentration of the substrate base is 10-200 mM, and a concentration of the substrate phosphate is 1-100 mM.
